# EUROPÄISCHE PATENTSCHRIFT

(11) **EP 1 642 609 B1**
(45) Veröffentlichungstag und Bekanntmachung des Hinweises auf die Patenterteilung: **24.10.2012**
(21) Anmeldenummer: 05090275.8
(22) Anmeldetag: 29.09.2005
(51) Int. Cl.: A61M 21/00

(54) **Augenvorsatz für Stimulationsgerät**
Eyeadapter for a stimulation device
Adaptateur pour les yeux à usage avec un dispositif de stimulation

(30) Priorität: 01.10.2004 DE 102004048982
(43) Veröffentlichungstag der Anmeldung: 05.04.2006
(73) Patentinhaber: Klose, Markus, 10175 Berlin (DE)
(72) Erfinder: Klose, Markus, 10175 Berlin (DE)
(74) Vertreter: Theobald, Andreas

(56) Entgegenhaltungen:
- US-A- 4 315 502
- US-A- 4 396 259
- US-A- 5 518 497
- US-A- 5 944 405
- US-A1- 2003 202 338

## Beschreibung

Die vorliegende Erfindung betrifft ein Stimulationsgerät zum Stimulieren des zentralen Nervensystems und der Gehirnströme eines Menschen mittels variierender Lichtsignale.

Es ist bekannt, dass das zentrale Nervensystem und die Gehirnströme eines Menschen auf optische und akustische Reize reagieren. Dies macht man sich in Stimulationsgeräten, auch Mentalsysteme oder Mind Machines genannt, zu Nutze, indem einem Menschen visuelle und/oder akustische Reize mit bestimmten Frequenzen zugeführt werden. Das Gehirn tendiert dann dazu, seine Gehirnwellen an die Frequenz der visuellen und/oder akustischen Reize anzupassen. Diese Anpassung wird auch Frequenz-Folgeprinzip oder Mind-Machine-Prinzip genannt. Der Effekt ähnelt dem beruhigenden Blick in ein flackerndes Lagerfeuer oder ein funkelndes Aquarium.

Auf dem Markt befindliche Stimulationsgeräte umfassen in der Regel eine Brille mit einem lichtundurchlässigen Brillenglas, auf dessen Innenseite LEDs angeordnet sind, die farbiges Licht auf die geschlossenen Augen abstrahlen. Zum Übertragen von Audioreizen umfassen die Geräte in der Regel zudem einen Kopfhörer. Derartige Stimulationsgeräte sind bspw. in US 5,409,445, in US 5,709,645, DE 38 23 402 A1 und in GB 1 422 959 beschrieben. Die DE 42 22 354 A1 beschreibt zudem ein Verfahren zum Stimulieren eines Menschen, in dem sich Lichtimpulse synchron zu einer Musik verändern.

Die US 5,518,497 und die US 4,396,259 beschrieben Augenvorsätze, bei denen Lichtquellen in Blickrichtung des entspannten Auges angeordnet sind. Zwischen den Lichtquellen und dem Auge befindet sich jeweils eine streuende Scheibe.

Die US 4,315,502 beschriebt einen Augenvorsatz mit einem von LEDs beleuchteten Diffusorring im Peripheriebereich des Augenvorsatzes. Die Beleuchtung führt dazu, dass ein Nutzer des Augenvorsatzes einen Lichtring wahrnimmt.

Die US 2003/0202338 A1 beschreibt eine Licht emittierende Anordnung, die die Form einer Auflage oder Decke ("pad or blanket") aufweisen kann und die beispielsweise zum Behandeln der Augen mit Licht Verwendung finden kann.

Aufgabe der vorliegenden Erfindung ist es, einen weiterentwickelten Augenvorsatz für ein Stimulationsgerät zum Stimulieren des zentralen Nervensystems und der Gehirnströme eines Menschen sowie ein weiterentwickeltes Stimulationsgerät zur Verfügung zu stellen.

Diese Aufgabe wird durch einen Augenvorsatz mit den in Anspruch 1 angegebenen Merkmalen sowie durch ein Stimulationsgerät mit den in Anspruch 8 angegebenen Merkmalen gelöst.

Der erfindungsgemäße Augenvorsatz in Form einer Brille weist mindestens drei verschiedenfarbige Lichtquellen auf, so dass durch Farbmischung Licht des gesamten Farbspektrums generiert werden kann. Als verschiedenfarbige Lichtquellen bieten sich hierbei rote, grüne und blaue Lichtquellen an. Um eine bessere Regulierung der Helligkeit und eine bessere Beeinflussung der erzeugbaren Lichtmuster zu ermöglichen, kann zudem eine weiße Lichtquelle vorhanden sein. Alternativ ist es auch möglich, eine Lichtquelle oder alle Lichtquellen als in der Farbe variierbare Lichtquellen auszugestalten.

Die Lichtquellen sind in dem erfindungsgemäßen Augenvorsatz derart an mindestens einer die Vorderseite mit der Rückseite verbindenden Seitenfläche einer in Form eines Brillenglases ausgebildeten transparenten Platte angeordnet, dass das Licht zwischen Vorder- und Rückseite in die Platte des Brillenglases hinein abgestrahlt wird. Das zwischen die Vorder- und Rückseite einer Platte eingestrahlte Licht wird in der Platte gestreut und gelangt so indirekt in das Auge bzw. die Augen des Trägers des Augenvorsatzes. Insbesondere wenn die mindestens eine Lichtquelle an der oberen Seitenfläche der transparenten Platte(n) angeordnet ist, entsteht der Eindruck eines natürlichen Lichteinfalls von oben. Die transparente Platte ist flächig ausgebildet. Die Größe ihrer Fläche und ihre Anordnung im Augenvorsatz bzw. ihre Integration in den Augenvorsatz sind derart aufeinander abgestimmt, dass das Sehfeld eines Auges oder beider Augen durch die Fläche weitgehend ausgefüllt wird. Die Beleuchtung des beleuchteten Elementes führt dann dazu, dass einem Benutzer des Augenvorsatzes der Eindruck entsteht, das über das beleuchtete Element indirekt zugeführte Licht komme aus allen Richtungen. Mit Hilfe dieses so genannten Ganzfeld-Effektes kann durch geeignete Lichtmuster auch der Eindruck eines virtuellen Raumes erweckt werden.

Als Lichtquellen kommen sowohl primäre als auch sekundäre Lichtquellen in Frage. Unter primären Lichtquellen sollen nachfolgend Licht emittierende Bauelemente, beispielsweise Leuchtdioden (LEDs) oder Glühlampen, zu verstehen sein. Sekundäre Lichtquellen sollen im Folgenden dagegen nicht selbst leuchtende Lichtquellen sein, denen Licht von einer entfernt angeordneten primären Lichtquelle zugeführt wird. Als Beispiel für eine sekundäre Lichtquelle sei das Austrittsende einer lichtleitenden optischen Faser genannt.

Von Bedeutung für die Erfindung ist insbesondere, dass das Licht der Lichtquelle die Augen nicht direkt erreicht, wenn diese sich im entspannten Zustand befinden, also geradeaus schauen. Die Lichtquelle soll daher insbesondere auch dann als so angeordnet, dass das Licht die Augen nicht direkt erreicht, angesehen werden, wenn das Licht die Augen im entspannten Zustand nicht direkt erreicht, in einem angespannten, verdrehten Zustand der Augen jedoch ein direkter Blick in die Lichtquelle möglich ist.

Dadurch, dass das Licht der Lichtquelle die Augen nur indirekt über das beleuchtete Element erreicht, sind die Augen nicht der vollen Intensität der Lichtquelle ausgesetzt. Der Augenvorsatz kann daher auch bei geöffneten Augen verwendet werden. Im Unterschied dazu sind die LEDs bei den meisten im Stand der Technik beschriebenen Brillen direkt vor den Augen angeordnet. Ein Verwenden der Brille bei geöffneten Augen ist daher wegen der hohen Intensität der Lichtquelle zumindest unangenehm, wenn nicht gar unmöglich. Die Intensitätsverringerung durch das beleuchtete optische Element kann durch diffuse Streuung oder diffuse Reflektion erreicht werden.

Es sei an dieser Stelle darauf hingewiesen, dass der erfindungsgemäße Augenvorsatz auch mit geschlossenen Augen verwendet werden kann. Alleine das Schließen der Augen kann bereits einen entspannten Zustand herbeiführen, der sich durch das Beleuchten der geschlossenen Augen verstärken lässt.

In der beschriebenen Ausgestaltung kann die Rückseite der Platte(n) lichtundurchlässig ausgestaltet sein. Außerdem können die Seitenflächen mit wenigstens einem Abschirmelement ausgestattet sein, das einem Lichtfall von außen, d.h. durch den Spalt zwischen der wenigstens einen Platte und dem Gesicht des Benutzers verhindert. In dieser Ausgestaltung braucht der erfindungsgemäße Augenvorsatz lediglich die Platte(n), das bzw. die an der Platte bzw. den Platten angeordnete(n) Abschirmelement(e) sowie die Halterung für die Platte(n) zu aufzuweisen.

Als Abschirmelement kann beispielsweise eine um die Seitenflächen herum angeordnete lichtundurchlässige Schürze, bspw. aus Gummi, vorhanden sein.

In der beschriebenen Ausgestaltung können insbesondere zwei transparente Platten, je eine für das linke und das rechte Auge, vorhanden sein. Auf diese Weise lässt sich besonders kostengünstig eine ergonomische Form der Platten realisieren, so dass der Augenvorsatz einen angenehmen Tragkomfort aufweist.

In allen Ausgestaltungen der vorliegenden Erfindung kann die Lichtquelle beispielsweise als Lichtdiode (primäre Lichtquelle) oder als Austrittsfläche eines Lichtleiters (sekundäre Lichtquelle) ausgebildet sein. Es ist jedoch auch möglich, in demselben Augenvorsatz sowohl primäre als auch sekundäre Lichtquellen zu verwenden.

Ein erfindungsgemäßes Stimulationsgerät umfasst einen erfindungsgemäßen Augenvorsatz und eine Lichtsignale generierende und auf die mindestens eine Lichtquelle einwirkende Steuereinheit. Um mittels des Frequenz-Folge-Prinzips Einfluss auf die Gehirnwellen nehmen zu können, kann die Steuereinheit insbesondere zum Herbeiführen von mit einer Frequenz zwischen 0,5 Hz und 40 Hz variierenden Lichtsignalen ausgestaltet sein. Insbesondere können die Lichtsignale rechteck-, dreieck- oder sinusförmige Signalverläufe aufweisen.

Wenn getrennte Lichtquellen für das linke und das rechte Auge vorhanden sind, kann die Steuereinheit derart ausgestalte sein, dass dem linken und dem rechten Auge immer identische Lichtsignale zugeführt werden. Mit anderen Worten, es werden sowohl die linke als auch die rechte Seite des Augenvorsatzes parallel angesteuert, also gekoppelt betrieben. Eine getrennte Ansteuerung ist aber auch möglich.

Um eine audiovisuelle Stimulation zu ermöglichen, kann das erfindungsgemäße Stimulationsgerät außerdem einen Kopfhörer zum Zuführen von Klangstrukturen umfassen.

Weitere Merkmale, Eigenschaften und Vorteile der vorliegenden Erfindung ergeben sich aus der nachfolgenden Beschreibung von Ausführungsbeispielen unter Bezugnahme auf die beiliegenden Figuren.
- Fig. 1: zeigt ein erstes Ausführungsbeispiel für den erfindungsgemäßen Augenvorsatz.
- Fig. 2: zeigt den Augenvorsatz des ersten Ausführungsbeispiels in einer perspektivischen Darstellung.
- Fig. 3: zeigt das beleuchtete Element des ersten Ausführungsbeispiels in einer schematischen Darstellung.
- Fig. 4: zeigt ein Beispiel für einen alternativen Augenvorsatz, der nicht Gegenstand der Erfindung ist.
- Fig. 5: zeigt das beleuchtete Element des Beispiels.
- Fig. 6: zeigt ein Detail aus Fig. 5.
- Fig. 7: zeigt ein Ausführungsbeispiel für das erfindungsgemäße Stimulationsgerät.

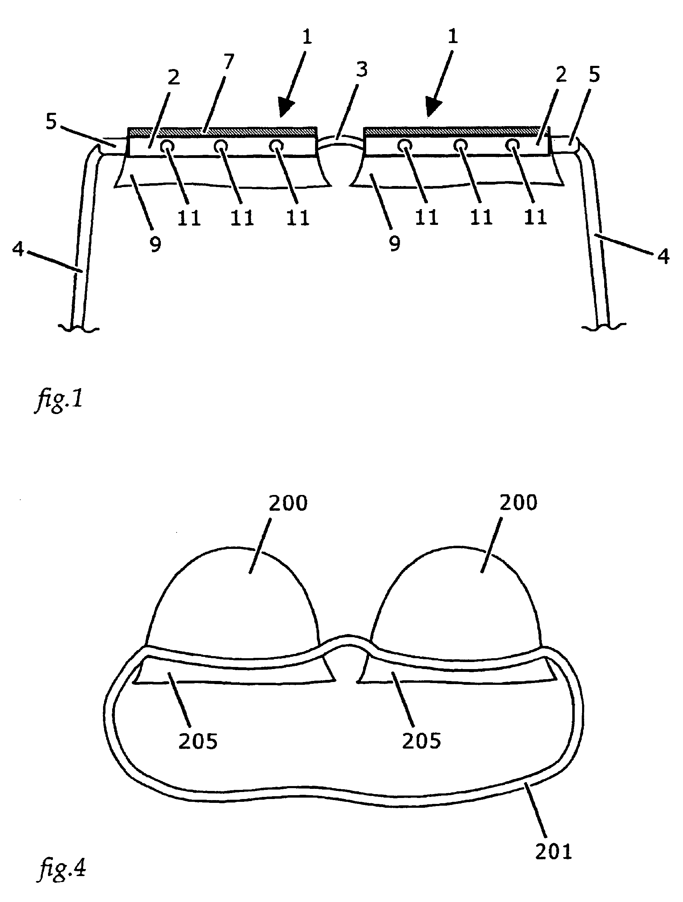

Ein erstes Ausführungsbeispiel für den erfindungsgemäßen Augenvorsatz ist in Fig. 1 dargestellt. Der Augenvorsatz hat die Form einer Brille mit zwei Brillengläsern 1, die über einen Nasenbügel 3 miteinander verbunden sind.

An den dem Nasenbügel 3 gegenüberliegenden Seiten der Brillengläser 1 sind Brillenbügel 4 über Scharniere 5 an den Gläsern 1 befestigt.

Die Rückseite der Gläser 1, das heißt die dem Auge abgewandte Seite der Gläser 1, ist mit einem lichtdichten Überzug 7 versehen. Außerdem ist jedes Brillenglas 1 an seinem Rand von einer lichtdichten Gummischürze 9 umgeben. Die Gummischürze 9 schließt beim Aufsetzen der Brille bündig mit dem Gesicht des Benutzers ab, so dass ein seitlicher Lichteinfall in die Augen unterdrückt wird. Selbstverständlich können auch andere Materialien als Gummi, beispielsweise ein lichtundurchlässiger Schaumstoff, verwendet werden, um die Brille gegen seitlichen Lichteinfall abzudichten.

Aufgrund der lichtdichten Überzüge 7 und der Gummischürzen 9 schließt die Brille nach dem Aufsetzen weitgehend lichtdicht ab.

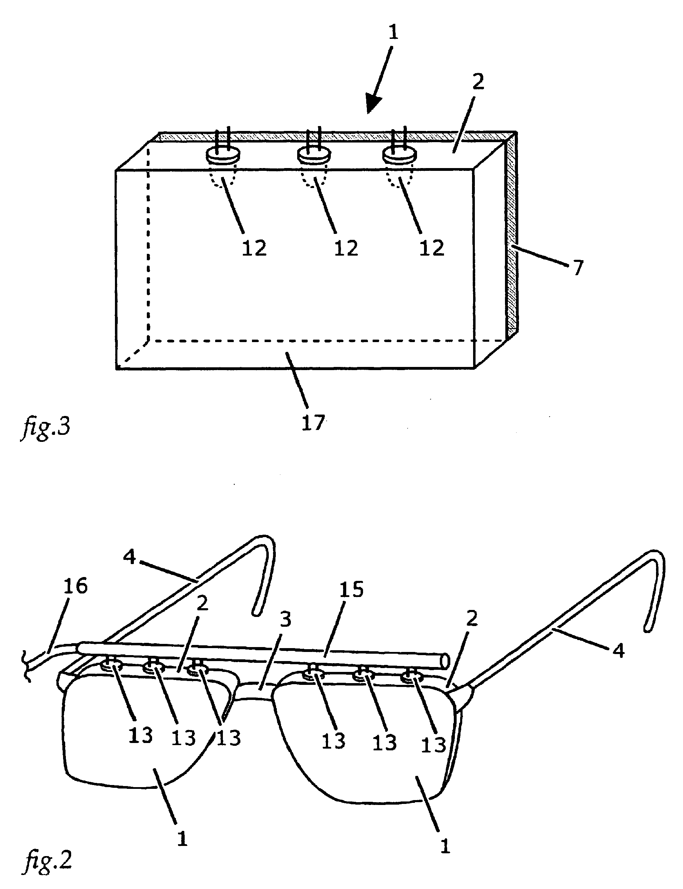

Die Oberseite 2 der Brillengläser ist mit Bohrungen 11 versehen, in die Leuchtdioden 12 (Fig. 3) eingebracht sind. Eine perspektivische Ansicht der Brille mit den Anschlüssen 13 von in die Bohrungen 11 eingebrachten Leuchtdioden 12 ist in Fig. 2 dargestellt. Die Leuchtdioden 12 werden über eine Anschlussleiste 15, welche über ein Kabel 16 mit einer in Fig. 2 nicht dargestellten Steuereinheit verbunden ist, mit Strom versorgt.

Die drei Leuchtdioden 12 unterscheiden sich im vorliegenden Ausführungsbeispiel in der Farbe des von ihnen abgestrahlten Lichtes. Eine der Leuchtdioden strahlt rotes Licht, eine weitere grünes Licht und die dritte blaues Licht ab. Mittels einer Farbmischung durch geeignetes Ansteuern der einzelnen Leuchtdioden 12 lassen sich so alle Farben des Spektrums generieren. Zusätzlich zu den drei farbigen Leuchtdioden 12 kann außerdem eine weiße Leuchtdiode vorhanden sein. Die weiße Leuchtdiode ermöglicht eine verbesserte Steuerung der Helligkeit sowie eine bessere Beeinflussung von Interferenzmustern.

Die Funktionsweise des Augenvorsatzes wird nachfolgend mit Bezug auf Fig. 3 beschrieben. Fig. 3 zeigt in einer stark schematisierten Darstellung ein Brillenglas 1 mit darin angeordneten Leuchtdioden 12. Die in den Bohrungen angeordneten Leuchtdioden 12 strahlen von der Oberseite 2 des Brillenglases aus Licht zwischen die Vorder- und die Rückseite des Brillenglases 1 ein. Das Brillenglas 1 ist derart ausgestaltet, dass das Licht zwischen der Vorderseite und der Rückseite in die Platte hinein geleitete wird, wo eine Streuung wenigstens eines Teils des eingestrahlten Lichtes in Richtung auf das Auge des Brillenträgers erfolgt.

Die Form des Brillenglases 1 und die Anordnung der Leuchtdioden 12 im Bereich der Oberseite 2 des Brillenglases 1 ist derart gewählt, dass bei geradeaus schauendem Auge kein von den Leuchtdioden 12 abgestrahltes Licht direkt das Auge erreichen kann. Es erreicht daher nur gestreutes Licht, also indirektes Licht der Leuchtdioden, das Auge. Dies führt u.a. dazu, dass die Strahlungsleistung der Leuchtdioden 12 auf eine größere Fläche verteilt wird und das Licht dem Betrachter daher weniger intensiv erscheint. Der Betrachter kann daher in das gestreute Licht schauen, ohne geblendet zu werden. Der erfindungsgemäße Augenvorsatz kann somit bei geöffneten Augen betrieben werden.

Die Ausdehnung der Brillengläser 1 ist außerdem so groß, dass die Vorderseiten 17 der Brillengläser 1 das gesamte Sichtfeld des Trägers weitgehend oder gar vollständig ausfüllen. Auf diese Weise kann gestreutes Lichte im Wesentlichen aus dem gesamten Gesichtsfeld in das Auge gelangen, so dass der Ganzfeld-Effekt realisiert werden kann. Die zum Erzielen des Ganzfeld-Effektes benötigte Größe der Brillengläser 1 hängt dabei vom Abstand der Brillengläser von den Augen ab.

Als Material für die Brillengläser eignen sich transparente Kunststoffplatten, beispielsweise oberflächenbehandeltes Acryl. Aber auch Glas ist grundsätzlich geeignet. Wenn oberflächenbehandeltes Acryl, bspw. satiniertes Acryl Verwendung findet, liegt die Oberflächenrautiefe vorteilhafterwiese im Bereich von 10 *µm* bis 100 *µm.* Liegt die Oberflächenrautiefe wesentlich unter 10 *µm,* ist der Streueffekt nicht groß genug, liegt sie dagegen wesentlich über 100 *µm,* so ist die für den Benutzer wahrnehmbare Helligkeit zu gering.

Anstatt direkt an Nasenbügel 3 und Brillenbügel 4 befestigt zu sein, können die Brillengläser 1 im ersten Ausführungsbeispiel auch in einem Rahmen eingefasst sein, an dem wiederum die Brillenbügel befestigt sind. Alternativ kann zum Verbinden der beiden Brillengläser miteinander auch ein vorzugsweise elastisches Band Verwendung finden. Ebenso ist es möglich, den Augenvorsatz mittels eines elastischen Bandes am Kopf zu halten. Es brauchen auch nicht unbedingt zwei Brillengläser vorhanden zu sein. Ein einziges, beide Augen überspannendes Brillenglas kann ebenso gut Verwendung finden.

Außerdem ist es auch möglich, einen Teil der Lichtquellen 12 oder alle Lichtquellen 12 an anderen Seitenflächen als der Oberseite 2 der Brillengläser 1 anzuordnen.

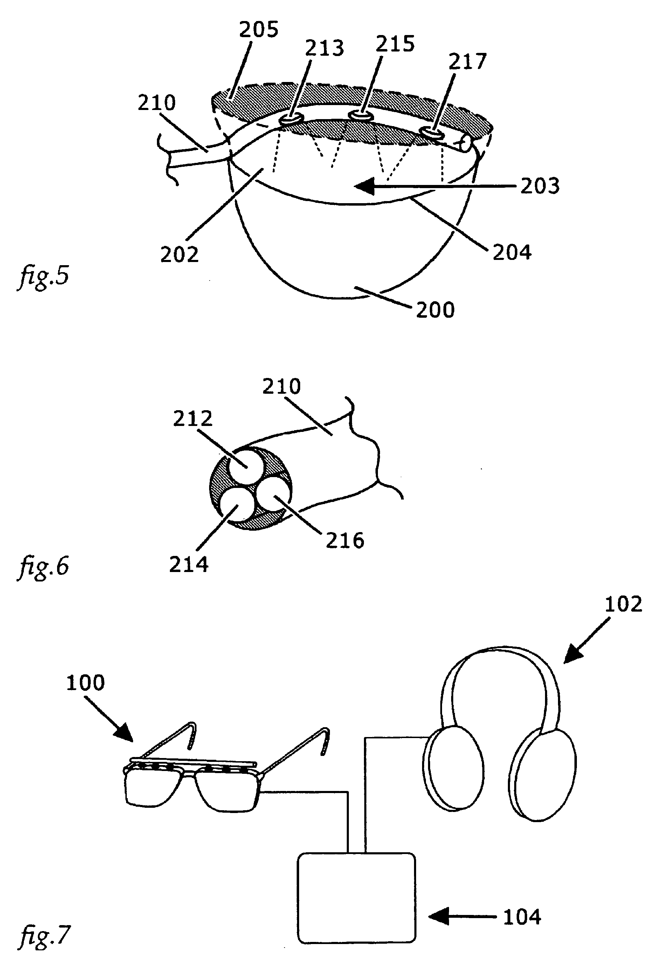

Ein erfindungsgemäßes Stimulationsgerät ist in Fig. 7 dargestellt. Das Stimulationsgerät umfasst eine Brille 100, wie sie mit Bezug auf die Figuren 1 bis 3 beschrieben worden ist. Daneben umfasst das Stimulationsgerät einen Kopfhörer 102 sowie eine Steuereinheit 104, die zur Ausgabe von Steuersignalen mit der Anschlussleiste 115 der Brille 100 in Signalverbindung steht. Die Steuereinheit 104 steht außerdem mit dem Kopfhörer 102 zur Ausgabe von Steuersignalen in Signalverbindung.

In der Steuereinheit 104 werden Steuersignale generiert, welche die Leuchtdioden der Brille 100 veranlassen variierende Lichtsignale auszugeben. Insbesondere werden die Leuchtdioden derart angesteuert, dass sie mit einer Frequenz zwischen 0,5 Hz und 40 Hz blinken. Die Steuereinheit 104 weist zudem eine Einstelleinrichtung auf, mit der die Frequenz im angegebenen Frequenzbereich eingestellt werden kann. Außerdem kann sie noch eine Möglichkeit zum Einstellen der Pulsform der Lichtimpulse beim Blinken umfassen, um beispielsweise rechteckförmige oder dreieckförmige Pulse oder auch Pulse in Sinuswellenform zu realisieren.

Vorzugsweise ist die Steuereinheit 104 derart ausgestaltet, dass die verschiedenfarbigen Leuchtdioden individuell angesteuert werden können. Aufgrund der Interferenz der einzelnen Farben der Leuchtdioden lassen sich dann kaleidoskopartige Strukturen in allen Farben des Spektrums darstellen.

Im vorliegenden Ausführungsbeispiel werden die Leuchtdioden 12 der beiden Brillengläser 1 in identischer Weise angesteuert, um den beiden Augen identische Lichtsignale zuzuführen. Es ist jedoch grundsätzlich auch möglich, die Leuchtdioden 12 der Brillengläser 1 getrennt anzusteuern, um für das rechte und das linke Auge verschiedene Lichtsignale zur Verfügung zu stellen. Beispielsweise können abwechselnd links/rechts Signale zugeführt werden. Es ist auch möglich, den Augen Lichtsignale in unterschiedlichen Farben zuzuführen, bspw. dem einen Auge rote Lichtsignale und dem anderen Auge grüne Lichtsignale.

Außer zum Ansteuern der Leuchtdioden dient die Steuereinheit 104 auch zum Ausgeben von Steuersignalen an die Lautsprecher des Kopfhörers 102. Die vom Kopfhörer 102 anhand der Steuersignale ausgegebenen Klangstrukturen oder Tonfolgen können mit den Lichtsignalen synchronisiert sein. Als Klangstrukturen bzw. Tonfolgen kommen Naturgeräusche, bspw. Wasserrauschen, Musikinstrumente, elektronische Klänge oder Klänge als Impulsfrequenzen in Frage.

Die Steuereinheit 104 kann eine Anzahl fest installierter Programme umfassen, die bspw. jeweils einen Zeitraum von 10 bis 60 Minuten überdecken. Zum Laden neuer Programme umfasst die Steuereinheit 104 eine Schnittstelle, die bspw. als Steckkartenplatz, als USB-Schnittstelle, als Infrarotschnittstelle oder als sonstige Schnittstelle ausgestaltet sein kann.

Die primäre Funktion des erfindungsgemäßen Stimulationsgerätes besteht in seiner entspannenden Wirkung. Es sind jedoch auch andere Anwendungen möglich. Als Beispiele seien an dieser Stelle therapeutische Anwendungen und Lernhilfen genannt. Für therapeutische Anwendungen ist es vorteilhaft, wenn das Stimulationsgerät auf einzelne bestimmte Farben und Frequenzen eingestellt werden kann, etwa auf hellgrün bei 10,5 Hz.

Ein Beispiel für einen alternativen Augenvorsatz, der nicht Gegenstand der Erfindung ist, ist in den Figuren 4 bis 6 dargestellt.

Der Augenvorsatz umfasst zwei in etwa halbkugelförmige hohle Elemente 200, die über ein elastisches Band 201 miteinander verbunden sind. Das elastische Band 201 dient einerseits als Einfassung für die halbkugelförmigen Elemente 200 und andererseits als Halteband zum Halten des Augenvorsatzes am Kopf eines Benutzers. Statt des Bandes kann jedoch auch ein Gestell mit Brillenbügeln vorhanden sein. Alternativ ist es auch möglich, die halbkugelförmigen Elemente 200 über ein Band oder einen Nasenbügel miteinander zu verbinden und die Brillenbügel direkt an den halbkugelförmigen Elementen 200 zu befestigen.

Ein halbkugelförmiges Element 200 ist in Fig. 5 im Detail dargestellt. Bei aufgesetztem Augenvorsatz befindet sich das halbkugelförmige Element 200 mit seiner Öffnung 203 vor dem Auge des Trägers. Der Radius der Öffnung 203 ist so gewählt, dass das halbkugelförmige Element 200 das Auge vollständig umschließt. Dadurch lässt sich der bereits erwähnte Ganzfeld-Effekt erzielen. Um einen seitlichen Lichteinfall in das Auge zu Unterbinden, ist am Rand 204 der Öffnung 203 eine elastische Schürze 205 angebracht, die sich über den gesamten Rand 204 erstreckt.

An der Innenseite des Randes 204 ist ein Faserbündel 210 angeordnet, das drei lichtleitende Fasern 212, 214, 216 (Fig. 6) umfasst. Die Fasern 212, 214, 216 enden jeweils in einer Austrittsfläche 213, 215, 217 am Rand 204 der Öffnung 203. Durch die Austrittsflächen tritt das durch die Fasern geleitete Licht in Richtung auf die Innenfläche 202 des halbkugelförmigen Elementes 200 aus. Die Innenfläche 202 ist diffus reflektierend ausgebildet und stellt eine konkav gewölbte Reflektionsfläche für das durch die Austrittsflächen 213, 215, 217 austretende Licht dar.

Aufgrund der Anordnung der Austrittsflächen 213, 215, 217 am Rand des halbkugelförmigen Elementes 200 - und damit am Rand der reflektierenden Fläche 202 - sind diese bei geradeaus blickendem Auge nicht direkt zu sehen, d.h. das austretende Licht kann das Auge nur indirekt durch Reflektion an der Reflektionsfläche 202 erreichen.

Die drei Austrittsflächen 213, 215, 217 stellen sekundäre Lichtquellen dar, die über die Lichtleiter 212, 214, 216 mit primären Lichtquellen (nicht dargestellt) verbunden sind. Vorzugsweise sind drei primäre Lichtquellen vorhanden, die Licht in verschiedenen Farben abgeben. Jede Austrittfläche ist dann über einen der Lichtleiter 212, 214, 216 mit einer dieser drei primären Lichtquellen verbunden, so dass die Austrittflächen 213, 215, 217 verschiedenfarbige sekundäre Lichtquellen darstellen. Mittels einer geeigneten Steuerung der primären Lichtquellen oder der Transmission durch die Lichtleiter 212, 214, 216 kann daher eine Farbmischung erfolgen. Die gleichfarbigen Austrittflächen der beiden Halbkugelförmigen Elemente können ihr Licht von derselben primären Lichtquelle empfangen. Alternativ kann jedoch auch jeder dieser Austrittsflächen eine eigene primäre Lichtquelle zugeordnet sein.

Im Betrieb des Augenvorsatzes werden die primären Lichtquellen derart betrieben, dass die Lichtabgabe der sekundären Lichtquellen variiert. Insbesondere kann eine pulsförmige Lichtabgabe mit Frequenzen zwischen 0,5 und 40 Hz stattfinden.

Obwohl die Austrittsflächen 213, 215, 217 im vorliegenden Ausführungsbeispiel am oberen Rand eines halbkugelförmigen Elementes 200 angeordnet sind, um einen Lichteinfall von oben zu erhalten, können die Austrittsflächen auch in anderen bereichen des Randes 204 angeordnet sein oder gleichmäßig über den Rand 204 verteilt sein. Es müssen auch nicht unbedingt genau drei Austrittsflächen vorhanden sein. Beispielsweise kann eine vierte Austrittsfläche über eine Lichtleitfaser mit einer weißes Licht abgebenden primären Lichtquelle verbunden sein. Schließlich ist es auch möglich, mehrere Austrittsflächen mit einer gemeinsamen primären Lichtquelle zu verbinden. Dies ist insbesondere dann von Interesse, wenn eine Vielzahl von ggf. gleichfarbigen Austrittsflächen entlang des Randes 204 der halbkugelförmigen Elemente 200 angeordnet werden sollen, bspw. um eine gleichmäßige Ausleuchtung der Reflektionsfläche 202 zu erzielen.

Am Rand 204 der halbkugelförmigen Elemente 200 können statt sekundären Lichtquellen auch primäre Lichtquellen, beispielsweise miniaturisierte Lichtdioden, angeordnet sein. Statt dem Faserbündel 210 verlaufen dann elektrische Zuleitungen entlang der Ränder 204 der halbkugelförmigen Elemente 200. Entsprechend können im ersten Ausführungsbeispiel sekundäre Lichtquellen statt der primären Lichtquellen an den Brillengläsern angeordnet sein. Auch eine Kombination aus primären und sekundären Lichtquellen ist in beiden Ausführungsbeispielen grundsätzlich möglich.

Selbstverständlich braucht der Augenvorsatz des Beispiels für den alternativen Augenvorsatz nicht notwendigerweise halbkugelförmige Elemente auszuweisen. Es genügt, wenn Elemente vorhanden sind, die eine konkav gewölbte Innenfläche als Reflektionsfläche aufweisen.

Der Augenvorsatz gemäß dem Beispiel für den alternativen Augenvorsatz kann auch anstelle der Brille 100 im erfindungsgemäßen Stimulationsgerät (Fig. 7) Verwendung finden.

## Patentansprüche

1. Augenvorsatz für ein Stimulationsgerät zum Stimulieren des Zentralnervensystems und der Gehirnströme eines Menschen mittels variierender Lichtsignale, der die Form einer Brille aufweist und derart ausgestaltet ist, dass er vor den Augen getragen die Augen gegen Lichteinfall von außen abschirmt und der mindestens drei verschiedenfarbiges Licht abgebende Lichtquellen (12) zum Abgeben der variierenden Lichtsignale umfasst, wobei
- die Lichtquellen (12) derart am Augenvorsatz angeordnet oder in diesen integriert sind, dass ihr Licht die Augen nicht direkt erreicht,
- wenigstens ein von der mindestens einen Lichtquelle (12) beleuchtetes Element derart in den Augenvorsatz integriert oder an diesem angeordnet ist, dass das Licht der Lichtquelle (12) die Augen über das wenigstens eine beleuchtete Element indirekt erreicht, wobei das beleuchtete Element wenigstens ein bei aufgesetztem Augenvorsatz vor einem Auge oder beiden Augen befindliche transparente Platte (1) mit einer dem Auge bzw. den Augen zugewandten Vorderseite (17) und einer dem Auge bzw. den Augen abgewandten Rückseite umfasst, wobei die transparente Platte (1) flächig ausgebildet ist und die Größe ihrer Fläche und ihre Anordnung im Augenvorsatz bzw. ihre Integration in den Augenvorsatz derart aufeinander abgestimmt sind, dass das Sehfeld eines Auges oder beider Augen bei aufgesetztem Augenvorsatz durch die Fläche weitgehend ausgefüllt wird., **dadurch gekennzeichnet, dass**
- die transparente Platte als Brillenglas (1) ausgebildet ist und
- die mindestens eine Lichtquelle (12) derart an mindestens einer die Vorderseite (17) mit der Rückseite verbindenden Seitenfläche (2) des Brillenglases (1) angeordnet ist, dass das Licht zwischen Vorder- und Rückseite in das Brillenglas (1) hinein abgestrahlt wird.

2. Augenvorsatz nach Anspruch 1,, **dadurch gekennzeichnet, dass** die Lichtquellen (12) an der oberen Seitenfläche (2) des Brillenglases (1) angeordnet ist.

3. Augenvorsatz nach einem der vorangehenden Ansprüche, **dadurch gekennzeichnet, dass** das Brillenglas (1) eine oberflächenbehandelte Acrylplatte ist.

4. Augenvorsatz nach Anspruch 3, **dadurch gekennzeichnet, dass** die oberflächenbehandelte Acrylplatte eine Oberflächenrautiefe im Bereich von 10 µm bis 100 µm besitzt..

5. Augenvorsatz nach einem der vorangehenden Ansprüche, **dadurch gekennzeichnet, dass** die Rückseite (7) des Brillenglases (1) mit einem lichtdichten Überzug versehen ist.

6. Augenvorsatz nach Anspruch 6, **dadurch gekennzeichnet, dass** die Seitenflächen des Brillenglases (1) mit einem Abschirmelement ausgestattet ist.

7. Stimulationsgerät mit einem Augenvorsatz (100) nach einem der vorangehenden Ansprüche und einer Lichtsignale generierenden und auf die mindestens eine Lichtquelle einwirkenden Steuereinheit (104).

8. Stimulationsgerät nach einem der Anspruch 7, **dadurch gekennzeichnet, dass** es außerdem einen Kopfhörer (102) zum Zuführen von Klangstrukturen umfasst.

## Claims

1. An ocular attachment for a stimulation device for stimulating the central nervous system and the brain currents of a human being by means of varying light signals, which is in the form of spectacles and so designed that worn in front of the eyes it screens the eyes from the incidence of light from the exterior and which includes at least three light sources (12) delivering differently colored light for delivery of the varying light signals, in which
- the light sources (12) are arranged on or integrated in the ocular attachment in such a way that their light does not directly reach the eyes,
- at least one element illuminated by the at least one light source (12) is integrated in or arranged on the ocular attachment in such a way that the light of the light source (12) reaches the eyes indirectly by way of the at least one illuminated element, in which the illuminated element includes at least one transparent plate (1) which when the ocular attachment is fitted in place is disposed in front of an eye or both eyes, having a front side (17) which faces towards the eye or eyes and a rear side which is remote from the eye or eyes, in which the transparent plate (1) is flat and the size of its surface and its arrangement in or its integration in the ocular attachment are matched to each other in such a way that the field of vision of an eye or both eyes when the ocular attachment is fitted in place is substantially filled by the surface, **characterised in that**
- the transparent plate (1) is in the form of spectacles glass and
- the at least one light source (12) is arranged at at least one side surface (2) of the transparent plate (1), the side surface connecting the front side (17) to the rear side, in such a way that the light is irradiated into the plate (1) between the front side and the rear side.

2. An ocular attachment as set forth in claim 1 **characterised in that** the light sources (12) are arranged at the upper side surface (2) of the spectacles glass (1).

3. An ocular attachment as set forth in any of the preceding claims **characterised in that** the spectacles glass (1) is a surface-treated acryl plate.

4. An ocular attachment as set forth in claim 3 **characterised in that** the surface-treated acryl plate has a depth of surface roughness in the range from 10 µm to 100 µm.

5. An ocular attachment as set forth in any of the preceding claims **characterised in that** the rear side (7) of the spectacles glass (1) is provided with a light-tight coating.

6. An ocular attachment as set forth in claim 6 **characterised in that** and that the side faces of the spectacles glass (1) are provided with a screening element.

7. A stimulation device comprising an ocular attachment (100) as set forth in any of the preceding claims and a control unit (104) which generates light signals and acts on the at least one light source.

8. A stimulation device as set forth in claim 7 **characterised in that** it also includes a headset (102) for the feed of sound structures.

## Revendications

1. Adaptateur pour les yeux à utiliser avec un appareil de stimulation, destiné à stimuler le système nerveux et l'activité électrique du cerveau d'une personne au moyen de signaux lumineux variables, lequel présente la forme de lunettes et est conçu de sorte que, porté devant les yeux, il protège contre l'incidence de la lumière de l'extérieur, et qui comprend au moins trois sources de lumière (12), qui émettent une lumière de différentes couleurs pour l'émission des signaux lumineux variés, sachant que
- les sources de lumière (12) sont agencées sur ou intégrées dans l'adapteur pour les yeux, de sorte que la lumière ne parvienne pas directement aux yeux,
- au moins un élément, éclairé par la source de lumière (12) au moins prévue, est.intégré dans l'adapteur pour les yeux ou agencé sur celui-ci, de sorte que la lumière de la sources de lumière (12) parvienne indirectement aux yeux par l'intermédiaire de l'élément éclairé au moins prévu, sachant que ledit élément éclairé est doté d'au moins une plaque transparente (1), qui, placée devant un oeil ou devant les deux yeux, quand l'adaptateur pour les yeux est mis en place, est dotée d'une face avant (17), orientée vers l'oeil ou vers les yeux, et d'une face arrière, orientée à l'opposé de l'oeil ou des yeux, sachant que ladite plaque transparente (1) est de conception plane, continue et que la grandeur de sa surface et son agencement sur l'adaptateur pour les yeux ou son intégration dans ledit adaptateur pour les yeux sont adaptés l'une à l'autre de sorte que, lorsque l'adaptateur pour les yeux est mis en place, le champ visuel d'un oeil ou des deux yeux se trouve sensiblement rempli par la surface,
**caractérisé en ce que**
- la plaque transparente est réalisée en tant que verre de lunettes (1) et que
- la source de lumière (12) au moins prévue est disposée sur au moins une face latérale (2) du verre de lunettes (1), qui relie la face avant (17) et la face arrière, de sorte que la lumière rayonne dans le verre de lunettes (1), entre la face avant et la face arrière.

2. Adaptateur pour les yeux selon la revendication 1, **caractérisé en ce que** les sources de lumière (12) sont disposées sur la face latérale, supérieure (2) du verre de lunettes (1).

3. Adaptateur pour les yeux selon l'une des revendications précédentes, **caractérisé en ce que** le verre de lunette (1) est une plaque acrylique, traitée superficiellement.

4. Adaptateur pour les yeux selon la revendication 3, **caractérisé en ce que** la plaque acrylique, traitée superficiellement possède une profondeur de rugosité superficielle, qui est située dans une plage de 10 µm à 100 µm.

5. Adaptateur pour les yeux selon l'une des revendications précédentes, **caractérisé en ce que** la face arrière (7) du verre de lunettes (1) est pourvue d'un revêtement étanche à la lumière.

6. Adaptateur pour les yeux selon la revendication 5, **caractérisé en ce que** les surfaces latérales du verre de lunettes (1) sont équipées d'un élément de protection.

7. Dispositif de stimulation avec un adaptateur pour les yeux (100) selon l'une des revendications précédentes et une unité de commande (104), qui génère des signaux lumineux et agit sur au moins une source de lumière.

8. Dispositif de stimulation selon la revendication 7, **caractérisé en ce qu'**il est doté, en outre, d'un casque d'écoute (102) pour l'émission de structures acoustiques.
